# EUROPÄISCHE PATENTSCHRIFT

(11) **EP 3 628 340 B1**
(45) Veröffentlichungstag und Bekanntmachung des Hinweises auf die Patenterteilung: **24.11.2021**
(21) Anmeldenummer: 19185747.3
(22) Anmeldetag: 11.07.2019
(51) Int. Cl.: A61L 27/30

(54) **IMPLANTAT UND VERFAHREN ZU DESSEN HERSTELLUNG**
IMPLANT AND METHOD OF MANUFACTURING SAME
IMPLANT ET SON PROCÉDÉ DE FABRICATION

(30) Priorität: 27.09.2018 DE 102018123874
(43) Veröffentlichungstag der Anmeldung: 01.04.2020
(73) Patentinhaber: DOT GmbH, 18059 Rostock (DE)
(72) Erfinder: BAUMANN, Axel, 18059 Rostock (DE); DEMPWOLF, Henry, 18059 Rostock (DE); KRAWUTSCHKE, Marcus, 18059 Rostock (DE)
(74) Vertreter: Prinz & Partner mbB

(56) Entgegenhaltungen:
- EP-A1- 1 916 007
- EP-A1- 1 923 079
- BRAIC M ET AL: "Synthesis and characterization of TiN, TiAIN and TiN/TiAIN biocompatible coatings", SURFACE AND COATINGS TECHNOLOGY, ELSEVIER BV, AMSTERDAM, NL, Bd. 200, Nr. 1-4, 1. Oktober 2005 (2005-10-01), Seiten 1014-1017, XP027609023, ISSN: 0257-8972 [gefunden am 2005-10-01]
- JÄGER MARCUS: "Oberflächenmodifikation von Implantaten, Teil 2", ORTHOPAEDE, SPRINGER VERLAG, BERLIN, DE, Bd. 47, Nr. 5, 12. April 2018 (2018-04-12) , Seiten 445-458, XP036494094, ISSN: 0085-4530, DOI: 10.1007/S00132-018-3560-5 [gefunden am 2018-04-12]

## Beschreibung

Die vorliegende Erfindung betrifft ein Implantat zum Einsetzen in einen menschlichen oder tierischen Körper, mit mindestens einem Substrat aus einem metallischen Werkstoff und einer auf dem Substrat gebildeten Oberfläche, die ganz oder teilweise mit einer Hartstoffbeschichtung versehen ist. Ferner betrifft die Erfindung ein Verfahren zur Herstellung des Implantats.

Implantate der eingangs beschriebenen Art und Verfahren zu deren Herstellung sind bekannt. Derartige Implantate kommen beispielsweise in Form von Hüft- und Kniegelenk-Endoprothesen zum Einsatz. Ein weiteres Anwendungsgebiet ist die Wirbelsäulenprothetik, dabei insbesondere die Bandscheibenprothetik.

Im Handel erhältlich sind Implantate aus einer Zirkon-Niob-Legierung, bei denen definierte Flächen in einem Wärmebehandlungsprozess in eine verschleißmindernde Zirkonoxidkeramik umgewandelt werden. Diese Keramikschicht wird auch als "Oxinium-Oberfläche" bezeichnet. Die so hergestellten Implantate sind jedoch nur eingeschränkt verwendbar und relativ teuer. Außerdem weisen Zirkon-Legierungen nur sehr schlechte tribologische Eigenschaften auf, so dass bei einem Schichtversagen ein sofortiger Austausch des Implantats erforderlich wird.

Die EP 1 916 007 zeigt ein medizinisches Implantat mit einem Metallsubstrat und einer auf dem Substrat gebildeten Beschichtung, die eine Zwischenschicht und eine äußere Schicht aus Aluminiumoxid umfasst. Das Implantat kann durch ein Verfahren hergestellt werden, bei dem eine Zwischenschicht aus einem anderen Material als Aluminiumoxid auf die Substratoberfläche aufgetragen wird und eine äußere Schicht aus Aluminiumoxid durch physikalische Abscheidung aus der Gasphase (PVD) auf mindestens einem Teil der Zwischenschicht abgeschieden wird. Als Zwischenschicht kann ein Material verwendet werden, das aus der aus Titanaluminiumnitrid (TiAIN), Chromaluminiumnitrid (CrAIN), Aluminiumnitrid (AIN), Titancarbonitrid (TiCN), Titannitrid (TiN), Chromoxid (Cr₂O₃), Titanaluminid (TiAl), Chromnitrid (CrN) und Kombinationen davon bestehenden Gruppe ausgewählt ist.

Die DE10 2006 039 329 B3 offenbart ein Implantat zum Einsetzen in einen menschlichen oder tierischen Körper, bei dem ein Teil der oder die ganze Gelenkfläche mit einer verschleißmindernden Hartstoffbeschichtung bedeckt ist. Die Hartstoffbeschichtung kann eine äußere Deckschicht, beispielsweise aus Zirkonnitrid (ZrN) und eine Zwischenschicht zum Vermindern von Spannungen zwischen der Hartstoffbeschichtung und dem Implantatmaterial umfassen. Die Zwischenschicht kann als Mehrschichtsystem mit wenigstens zwei unterschiedlichen Schichten gebildet sein, die abwechselnd übereinanderliegen. Beispielsweise kann eine der beiden Schichten eine Chromnitrid-(CrN)-Schicht sein und die andere der beiden unterschiedlichen Schichten eine Chromcarbonitrid-(CrCN)-Schicht, wobei vorzugsweise ein Schichtsystem aus fünf Schichten entsteht, dessen äußere Schichten durch Chromnitrid-(CrN)-Schichten gebildet sind. Alternativ können als unterschiedliche Schichten auch Titannitrid-(TiN)-Schichten und Titancarbonitrid-(TiCN)-Schichten vorgesehen sein.

Die Beschichtungen sollen den Austritt von Metallionen wie beispielsweise Kobalt-, Chrom-, Molybdän- und/oder Nickel-Ionen aus dem Implantatmaterial reduzieren oder verhindern. Dadurch soll eine Korrosion des Implantats oder ein Verschleiß durch Abplatzen der Hartstoffbeschichtung verringert werden. Durch die Beschichtung kann außerdem die Verträglichkeit des Implantats verbessert und das durch den Austritt von Metallionen aus dem Implantatmaterial verursachte Auftreten allergischer Reaktionen verhindert werden.

Aus der EP 2 569 022 B1 ist ein Substrat für Gelenke von orthopädischen Implantaten bekannt, wobei mindestens eine der Gleitflächen aus NE-Material, insbesondere aus Kobalt-, Chrom-, Molybdänlegierungen, gebildet ist und eine Beschichtung aus Nitridschichten aufweist. Die Beschichtung enthält Niobnitrid-Nanoschichten und Chromnitrid-Nanoschichten und ist durch eine Chromnitrid-Mikroschicht geschützt. Die die Niobnitrid-Nanoschichten können sich mit den Chromnitrid-Nanoschichten abwechseln.

Die WO 2012/003899 A1 betrifft ein Medizinprodukt mit einer auf einen Grundkörper aufgebrachten antibakteriellen Hartstoffbeschichtung mit Biozid. Diese Hartstoffbeschichtung umfasst mindestens eine innere Schicht und eine äußere Schicht wobei die Biozidkonzentration in der äußeren Schicht im Wesentlichen konstant und größer als die Biozidkonzentration in der inneren Schicht ist und die Biozidkonzentration in der inneren Schicht größer oder gleich 0.2 at% beträgt. Im Ausführungsbeispiel sind silberdotierte TiN-Schichten offenbart.

Braic, M. et al., "Synthesis and characterization of TiN, TiAIN and TiN/TiAIN biocomptabile coatings", Surface & Coatings Technology 200, 2005, S.1014-1017 offenbart medizinische Implantate mit einem Metallsubstrat und einer Beschichtung mit Monoschichten aus TiN oder TiAIN. Alternativ wird eine Multilayerschicht mit TiN/TiAIN-Wechselschichten eingesetzt. Die einzelnen Wechselschichten weisen eine Schichtdicke von etwa 5 bis 630 nm auf. Die Gesamtdicke der Beschichtung beträgt etwa 3,8 µm.

Die EP 1 923 079 A zeigt medizinische Implantate mit einem metallischen Substrat und einer keramischen Beschichtung auf dem metallischen Substrat. Die keramische Beschichtung kann aus einer Reihe von Hartstoffen ausgewählt werden, darunter TiN, TiNbN und TiAIN. TiN und TiAIN werden als geeignete Materialien für Monoschichten beschrieben

Die bekannten Beschichtungen von Implantaten erfordern jedoch Multischichtstrukturen und sind daher in der Herstellung relativ teuer. Die Bereitstellung einer Vielzahl von unterschiedlichen Schichten kann außerdem zu einer Lokalelementbildung sowie zu unerwünschten mechanischen Spannungen zwischen der Beschichtung und dem Implantatmaterial und/oder zu inneren Spannungen in der Hartstoffbeschichtung führen, wodurch die Haftfestigkeit der Beschichtung beeinträchtigt wird. Beschichtungen mit einer Außenschicht aus Aluminiumoxid sind zudem relativ rau und neigen zu einem vorzeitigen Verschleiß eines Gelenkpartners.

Die Abscheidung von TiAIN-Schichten auf einem Substrat, insbesondere auf einem Hartmetallsubstrat, unter Verwendung von Verfahren der physikalischen Abscheidung aus der Gasphase (PVD-Verfahren) ist im Stand der Technik umfangreich beschrieben. Hierzu kann beispielsweise auf die DE 10 2012 107 129 A1, die EP 1 273 679 A1 und die DE 196 14 557 A1 verwiesen werden.

Es ist Aufgabe der vorliegenden Erfindung, ein Implantat der eingangs beschriebenen Art mit einer kostengünstigen Verschleißschutzschicht bereitzustellen. Insbesondere soll die Beschichtung eine gute Haftfestigkeit am Implantatmaterial aufweisen und eine ausreichende Lebensdauer des Implantats gewährleisten.

Diese Aufgabe wird durch ein Implantat gemäß Anspruch 1 gelöst. Weitere vorteilhafte Ausführungsformen sind in den Unteransprüchen angegeben, die wahlweise miteinander kombiniert werden können.

Das erfindungsgemäße Implantat zum Einsetzen in einen menschlichen oder tierischen Körper umfasst mindestens ein Substrat aus einem metallischen Werkstoff und eine auf dem Substrat gebildete Oberfläche. Die Oberfläche ist ganz oder teilweise mit einer Hartstoffbeschichtung versehen. Das Implantat ist dadurch gekennzeichnet, dass die Hartstoffbeschichtung eine durch ein PVD-Verfahren aufgebrachte Grundschicht aus TiN oder TiNbN und eine auf der Grundschicht durch ein PVD-Verfahren aufgebrachte Außenschicht aus TiAIN umfasst, wobei die Grundschicht eine Schichtdicke in einem Bereich von 1 bis 5 µm und die TiAIN-Außenschicht eine Schichtdicke von 1 bis 6 µm aufweist.

Insbesondere kann die Substratoberfläche mindestens teilweise in Form einer künstlichen Gelenkfläche ausgebildet sein, wobei die Gelenkfläche ganz oder teilweise, vorzugsweise vollständig mit der Hartstoffbeschichtung versehen sein kann.

Ferner betrifft die Erfindung ein Verfahren zur Herstellung des Implantats gemäß Anspruch 11 und die Verwendung des nach dem Verfahren hergestellten Implantats als Hüftgelenkprothese, Kniegelenkprothese, Sprunggelenkprothese oder Schultergelenkprothese, Wirbelkörperersatzimplantat oder Bandscheibenprothese.

Eine verschleißfeste Hartstoffbeschichtung in Form eines Zweischichtsystems mit einer Grundschicht aus TiN oder TiNbN und einer direkt an die Grundschicht angrenzenden Außenschicht aus TiAIN lässt sich unter Verwendung des PVD-Verfahrens schnell und kostengünstig herstellen. Gleichzeitig können durch das erfindungsgemäße Schichtsystem die mechanischen Spannungen zwischen der Hartstoffbeschichtung und dem Substrat und/oder innere Spannungen in der Hartstoffbeschichtung vermindert werden.

Die TiAIN-Schicht liegt erfindungsgemäß als Außenschicht der Hartstoffbeschichtung vor. Auf der TiAIN-Schicht ist somit keine weitere Schicht vorgesehen. Insbesondere steht die TiAIN-Schicht in unmittelbarem Kontakt mit Körpergewebe und/oder einer Anlagefläche auf einem Gelenkpartner.

Das Zweischichtsystem aus TiNbN und TiAIN bzw. TiN und TiAIN stellt eine hervorragende Barriere für den Durchtritt von Metallionen aus dem Substrat in das Körpergewebe dar. Außerdem kann die TiAIN-Außenschicht als glatte und defektarme Fläche mit einer äußerst niedrigen Oberflächenrauheit hergestellt werden, so dass ein Verschleiß durch Abrieb der TiAIN-Schicht oder der an die TiAIN-Schicht angrenzenden Anlagefläche des Gelenkpartners reduziert wird. Dadurch gelangen weniger fremde Partikel in den Körper des Implantatträgers und die Gefahr von Allergien kann deutlich reduziert werden. Gleichzeitig wirkt die Hartstoffbeschichtung als Korrosionsschutz für die beschichtete Gelenkfläche.

Die TiAIN-Außenschicht weist zudem eine hervorragende Benetzbarkeit für Körperflüssigkeit auf. Dadurch werden die tribologischen Eigenschaften der Gelenkfläche günstig beeinflusst.

Das erfindungsgemäße Schichtsystem zeigt zudem eine sehr gute Haftfestigkeit sowohl am Substrat als auch innerhalb des Schichtsystems. Dadurch ist die Gefahr eines Versagens der Beschichtung durch Abplatzen unter Belastung deutlich verringert.

Aufgrund der hohen Härte der TiAIN-Außenschicht kann die Beschichtung in einer niedrigen Schichtdicke von nur wenigen Mikrometern hergestellt werden. Auf diese Weise können Herstellungskosten eingespart werden, ohne die Leistungsfähigkeit des Implantats zu beeinträchtigen. Außerdem indiziert eine hohe Schichthärte auch eine Beständigkeit der Gelenkfläche gegenüber Dreikörperverschleiß und eine bessere Kratzfestigkeit.

Gemäß einer bevorzugten Ausführungsform weist die TiAIN-Außenschicht eine Zusammensetzung von Ti₁₋ₓAlₓN mit 0,2 ≤ x ≤ 0,8 auf, bevorzugt von 0,5 ≤ x ≤ 0,7, jeweils mit Ausnahme von unvermeidbaren Verunreinigungen. Eine TiAIN-Schicht mit der beschriebenen Zusammensetzung lässt sich leicht durch PVD-Verfahren herstellen. Die TiAIN-Schicht weist eine gute Haftfestigkeit an der TiNbN-Grundschicht und eine hohe Härte auf.

Bevorzugt ist die TiAIN-Schicht eine monolithische Schicht. Monolithisch bedeutet, dass innerhalb der TiAIN-Schicht keine Wechselschichten mit unterschiedlicher Schichtzusammensetzung an den jeweiligen Grenzflächen vorliegen. Die TiAIN-Schicht kann jedoch einen Aluminium-Gradienten mit einem nach außen, von der Substratoberfläche in Richtung auf die Außenfläche des Implantats, zunehmenden Aluminium-Anteil aufweisen, um die Anhaftung an der TiN- oder TiNbN-Grundschicht zu verbessern. Durch einen höheren Aluminium-Anteil an der Außenfläche des Implantats kann ferner die Härte und Verschleißfestigkeit der TiAIN-Schicht noch verbessert werden.

Bevorzugt ist die Grundschicht eine TiNbN-Schicht, besonders bevorzugt eine monolithische Schicht, mit einer Zusammensetzung von Ti₁₋ₓNbₓN und 0,1 ≤ x ≤ 0,4, mit Ausnahme von unvermeidbaren Verunreinigungen.

Das Nb in der TiNbN-Schicht kann ohne Beeinträchtigung der Schichteigenschaften teilweise durch Ta ersetzt sein, vorzugsweise in einem Anteil von bis zu 30 at%, vorzugsweise bis zu 10 at% und besonders bevorzugt bis zu 5 at%. Es ist bekannt, das Nb und Ta miteinander vergesellschaftet sind und eine vollständige Trennung der Elemente wegen der sehr ähnlichen Eigenschaften von Nb und Ta nur mit hohen Kosten zu erreichen ist. Bevorzugt liegen Ti und Nb im eingesetzten TiNb-Target jedoch als Reinelemente vor, mit Ausnahme von unvermeidbaren Verunreinigungen.

Die TiNbN-Schicht ist bevorzugt direkt auf der Gelenkfläche des metallischen Substrats aufgebracht. Direkt bedeutet, dass zwischen der Gelenkfläche und der TiNbN-Schicht keine weiteren funktionalen Schichten vorhanden sind, mit Ausnahme einer bis zu 500 nm dicken Anbindungsschicht aus Titan oder einer Titanlegierung, die eine Anhaftung der TiNbN-Schicht an dem metallischen Substrat begünstigt. Die Bereitstellung solcher Anbindungsschichten ist in PVD-Verfahren allgemein bekannt.

Gemäß einer bevorzugten Ausführungsform besteht die Hartstoffbeschichtung daher aus der Grundschicht, der TiAIN-Außenschicht und wahlweise der Anbindungsschicht aus Titan oder einer Titanlegierung.

Das Substrat kann aus jedem metallischen Werkstoff gebildet sein, der zur Herstellung von Implantaten geeignet ist, die in den menschlichen oder einen tierischen Körper eingebracht werden. Bekannte geeignete Werkstoffe sind beispielsweise Stahl, Titan und Titanlegierungen sowie Kobalt oder Kobaltlegierungen.

Vorzugsweise enthält der Werkstoff Kobalt oder ist eine Kobalt-Legierung. Vorteilhaft ist es, wenn die Kobalt-Legierung eine Kobalt-Chrom-MolybdänLegierung ist. Vorzugsweise handelt es sich dabei um eine CoCr29Mo6-Legierung.

Geeignete Titan-Legierungen umfassen, ohne darauf beschränkt zu sein, eine Titan-Aluminium-Vanadium-Legierung wie Ti-3Al-2,5V oder Ti-6Al-4V.

Ein als Substratmaterial für Implantate geeigneter Stahl ist beispielsweise Edelstahl 1.4301 (X5CrNi18-10), 1.4404 (X2CrNiMo18-14-3) und 1.4435 (X2CrNiMo18-14-3).

Die Gesamtdicke der Hartstoffbeschichtung beträgt vorzugsweise von 2 bis 10 µm, bevorzugt von 3 bis 8 µm und besonders bevorzugt von 4 bis 7 µm. Die Schichtdicke der TiN- oder TiNbN-Grundschicht liegt in einem Bereich von 1 bis 5 µm, bevorzugt von 1 bis 4 µm. Die TiAIN-Außenschicht weist eine Schichtdicke von 1 bis 6 µm auf, besonders bevorzugt von 2 bis 5 µm.

Trotz der vergleichsweise geringen Schichtdicke weist die Hartstoffbeschichtung eine gute Verschleißfestigkeit auf. Beschichtungen mit einer höheren Schichtdicke sind weniger stabil und neigen zum Abplatzen von dem Substrat.

Das Aufbringen der Hartstoffbeschichtung im PVD-Verfahren ermöglicht die Bereitstellung von Schichten mit geringer Oberflächenrauheit und günstigen tribologischen Eigenschaften. Bevorzugt liegt die mittlere Oberflächenrauheit Ra unterhalb von 0,05 µm, vorzugsweise unterhalb von 0,03 µm, und besonders bevorzugt in einem Bereich von 0,01 µm bis 0,03 µm. Die Messung der mittleren Oberflächenrauheit Ra erfolgt nach DIN EN ISO 4287.

Zur weiteren Glättung der Oberfläche des Implantats nach dem Abscheiden der Schichten eignen sich bekannte Polierverfahren wie Schleifen oder Bürsten mit entsprechend harten und feinen Materialien. Durch geeignete Wahl der Abscheideparameter im PVD-Verfahren kann jedoch eine niedrige mittlere Oberflächenrauheit Ra bereits unmittelbar nach Abscheidung der TiAIN-Schicht erhalten werden. Daher kann ein nachträgliches Glätten der Oberfläche entfallen.

Die Verschleißfestigkeit der Hartstoffbeschichtung ist gegenüber den herkömmlichen Implantatbeschichtungen mit einer TiNbN-Außenschicht wesentlich verbessert. Besonders bevorzugt weist die Hartstoffbeschichtung mit der TiAIN-Außenschicht eine Mikrohärte HV nach Vickers von 3200 bis 3600 HV (Prüfkraft 0,080 N aus Martenshärte) auf, die etwa um 40% höher liegt als die Härte einer TiNbN-Schicht.

Die Haftfestigkeit der Hartstoffbeschichtung kann in einem Rockwelltest ermittelt werden. Beim Rockwelltest wird ein Diamantkegel mit definierter Kraft in die Schichtoberfläche eingedrückt. In der Umgebung des Härte-Eindruckes ist die Schicht geschädigt, was sich im Mikroskop als Rissnetzwerk bzw. als Schichtausbrüche im Randbereich des Eindruckes erkennen lässt. Die Auswertung der Abplatzungen um den Eindruck herum kann durch Anwendung der DIN EN ISO 26443 mit digitaler Bildauswertung der abgeplatzten Flächenanteile geschehen. Die Haftfestigkeit der Hartstoffbeschichtung im Rockwelltest ergibt nach DIN/ISO 26443 maximal Klasse 1.

Gemäß einer bevorzugten Ausführungsform der Erfindung kann vorgesehen sein, dass das Implantat ein künstliches Hüft- oder Kniegelenk, ein künstliches Sprunggelenk, ein künstliches Schultergelenk, ein Wirbelkörperersatzimplantat oder eine künstliche Bandscheibenprothese ist.

Bevorzugt ist die Substratoberfläche mindestens teilweise in Form einer künstlichen Gelenkfläche ausgebildet wobei die Gelenkfläche ganz oder teilweise, vorzugsweise vollständig mit der Hartstoffbeschichtung versehen ist. Die Gelenkfläche kann beispielsweise eine Gelenkkugel oder eine Gelenkpfanne einer Hüftgelenkprothese, eine künstliche Kondyle, eine Tibiaplatte einer Kniegelenkprothese oder ein Anlageelement einer Bandscheibenprothese bilden.

Ein Verfahren zur Herstellung des erfindungsgemäßen Implantats zum Einsetzen in einen menschlichen oder tierischen Körper umfasst die Bereitstellung eines Substrats aus einem metallischen Werkstoff, wobei das Substrat eine Oberfläche aufweist, die mindestens teilweise in Form einer künstlichen Gelenkfläche ausgebildet werden kann, und das Aufbringen einer Hartstoffbeschichtung auf die ganze oder einen Teil der Oberfläche mittels eines PVD-Verfahrens. Das erfindungsgemäße Verfahren ist dadurch gekennzeichnet, dass das Aufbringen der Hartstoffbeschichtung dadurch erfolgt, dass eine Grundschicht aus TiN oder TiNbN auf die Oberfläche aufgebracht wird, und anschließend eine TiAIN-Schicht auf die Grundschicht aufgebracht wird, wobei die TiAIN-Schicht die Außenschicht der Hartstoffbeschichtung bildet.

Durch das Aufbringen der Hartstoffbeschichtung mittels eines PVD-Verfahrens kann ein besonders gutes Anhaften der Hartstoffbeschichtung am Substrat erreicht werden. Das Aufbringen der TiN- oder TiNbN-Grundschicht und der TiAIN-Außenschicht erfolgt bevorzugt ohne Unterbrechung in einem einzigen Beschichtungszyklus.

Bevorzugt wird eine TiNbN-Grundschicht unter Verwendung eines Ti-Nb-Targets mit 70 at% Titan und 30 at% Niob auf die Gelenkfläche des Substrats aufgebracht.

Das Aufbringen der TiAIN-Außenschicht kann beispielsweise unter Verwendung eines Ti-Al-Targets mit 33 at% Titan und 67 at% Aluminium erfolgen.

Das PVD-Verfahren kann als Magnetronsputtern, Lichtbogenverdampfen (Arc-PVD), Ionenplattierung, Elektronenstrahlverdampfung oder Laserablation durchgeführt werden. Bevorzugte PVD-Verfahren umfassen gepulstes und ungepulstes Magnetronsputtern, HF-Magnetronsputtern und Wechselstrom-Magnetronsputtern sowie gepulstes und ungepulstes Lichtbogenverdampfen. Zur Erzeugung von Nitriden wird dem Reaktionsraum des PVD-Verfahrens Stickstoff zugeführt. PVD-Apparaturen zum Aufbringen von Hartstoffbeschichtungen sind kommerziell erhältlich.

Die nachfolgende Beschreibung bevorzugter Ausführungsformen der Erfindung dient der näheren Erläuterung und soll nicht in einem einschränkenden Sinn verstanden werden.

Erfindungsgemäße Implantate zum Einsetzen in einen menschlichen oder tierischen Körper können beispielsweise in Form von Kniegelenkprothesen, Hüftgelenkprothesen, Sprunggelenkprothesen, Schultergelenkprothesen oder Bandscheibenprothesen ausgebildet sein. Diese Liste ist jedoch nicht abschließend.

Das Implantat umfasst mindestens ein Substrat aus einem metallischen Werkstoff und eine auf dem Substrat gebildete Oberfläche, die in Form einer künstlichen Gelenkfläche ausgebildet sein kann. Die Oberfläche, insbesondere die Gelenkfläche, ist ganz oder teilweise, bevorzugt vollständig, mit einer Hartstoffbeschichtung versehen.

Die Hartstoffbeschichtung umfasst erfindungsgemäß eine auf der Substratoberfläche durch ein PVD-Verfahren aufgebrachte Grundschicht aus TiN oder TiNbN und eine auf der Grundschicht durch ein PVD-Verfahren aufgebrachte Außenschicht aus TiAIN. Vorzugsweise besteht die Hartstoffbeschichtung aus der TiN- oder TiNbN-Grundschicht und der TiAIN-Außenschicht. Wahlweise kann eine Anbindungsschicht aus Titan oder einer Titanlegierung zwischen dem Substrat und der TiN- oder TiNbN-Grundschicht vorgesehen sein.

### Herstellung einer erfindungsgemäßen Hartstoffbeschichtung

Im hier beschriebenen Ausführungsbeispiel wurde ein Substrat aus einer Kobaltlegierung CoCr29Mo6 mit der erfindungsgemäßen Hartstoffbeschichtung versehen.

Auf der Substratoberfläche wurde zunächst eine 0,5 µm dicke TiNb-Anbindungsschicht durch Lichtbogenverdampfen von einem Ti-Nb-Mischtarget abgeschieden. Auf diese Anbindungsschicht wurde ebenfalls durch Lichtbogenverdampfen eine erfindungsgemäße Hartstoffbeschichtung mit einer Gesamtschichtdicke von 5,5 µm abgeschieden.

Die Abscheidung der TiNbN-Grundschicht erfolgte von einem Ti-Nb-Mischtarget mit einem Verhältnis Ti:Nb = 70:30 (at%) bei einer Substratspannung (Bias) von 100 bis 200 V und einem Stickstoffdruck von 2 bis 4 Pa. Die Abscheidungstemperatur betrug etwa 400 bis 500 °C. Es wurde eine TiNbN-Schicht mit einer Schichtdicke von 1 µm erhalten.

Direkt auf der TiNbN-Schicht wurde eine TiAIN-Schicht durch Lichtbogenverdampfen von einem Ti-Al-Mischtarget mit einem Verhältnis Ti:AI = 45:55 at% abgeschieden. Die Substratspannung lag zwischen 50 und 130 V bei einem Stickstoffdruck von 1 bis 4 Pa Stickstoff. Die Abscheidetemperatur lag bei etwa 400 bis 500 °C. Es wurde eine TiAIN-Schicht mit einer Schichtdicke von 5 µm erhalten

### Herstellung einer Vergleichsbeschichtung

Auf der Oberfläche eines Substrats aus CoCr29Mo6 wurde durch Lichtbogenverdampfen eine monolithische TiNbN-Schicht in einer Schichtdicke von 5,5 µm hergestellt. Es wurde ein Ti-Nb-Mischtarget mit einem Verhältnis Ti:Nb = 70:30 (at%) bei einer Substratspannung von 100 bis 200 V und einem Stickstoffdruck von 2 bis 4 Pa verwendet. Die Abscheidetemperatur betrug etwa 400 bis 500 °C.

An den so erhaltenen Beschichtungen wurden verschiedene Messungen zur Ermittlung der Oberflächenrauheit, der Schichtdicke, der Härte und der Haftfestigkeit durchgeführt. Die Ergebnisse sind in der nachfolgenden Tabelle dargestellt.

**Tabelle: Schichteigenschaften**

| Parameter | Verfahren | TiNbN-TiAIN | TiNbN |
|---|---|---|---|
| Mittlere Rauheit Ra | Taktile Messung nach DIN EN ISO 4287 | 0,02 µm | ≤ 0,05 µm |
| Schichtdicke | Kalottenschliff gemäß DIN EN 1071-2 | 5,3 µm | 4,5 µm |
| Härte | Registrierende Mikrohärtemessung nach Vickers (DIN EN ISO 14577-4; Prüfkraft 0,080 N) | 3384 HV | 2400 HV |
| Haftfestigkeit | Rockwell Test (DIN EN ISO 26443) | Max. Klasse 1 | Max. Klasse 1 |
| Adhäsive Versagenslast | Scratchtest Lc2 (DIN EN 1071-3) | 49,23 N | 49,30 N |

Durch das erfindungsgemäße Zweischichtsystem gelingt es, eine Hartstoffschicht für Implantate mit guten Verschleißschutzeigenschaften kostengünstig herzustellen, die auch den hohen Anforderungen an Gelenkprothesen mit Bezug auf tribologische und mechanische Eigenschaften erfüllt. Die einzelnen Schichten besitzen Barrierefunktion und zeigen gleichzeitig eine hohe Härte und eine hohe Haftfestigkeit. Dadurch kann die Lebensdauer des beschichteten Implantats verbessert werden.

## Patentansprüche

1. Implantat zum Einsetzen in einen menschlichen oder tierischen Körper, das mindestens ein Substrat aus einem metallischen Werkstoff und eine auf dem Substrat gebildete Oberfläche umfasst, wobei die Oberfläche ganz oder teilweise mit einer Hartstoffbeschichtung versehen ist, **dadurch gekennzeichnet, dass** die Hartstoffbeschichtung eine auf der Oberfläche durch ein PVD-Verfahren aufgebrachte Grundschicht aus TiN oder TiNbN und eine auf der Grundschicht durch ein PVD-Verfahren aufgebrachte Außenschicht aus TiAIN umfasst, wobei die Grundschicht eine Schichtdicke in einem Bereich von 1 bis 5 µm und die TiAIN-Außenschicht eine Schichtdicke von 1 bis 6 µm aufweist.

2. Implantat nach Anspruch 1, **dadurch gekennzeichnet, dass** die Oberfläche des Substrats mindestens teilweise in Form einer künstlichen Gelenkfläche ausgebildet ist, wobei die Gelenkfläche ganz oder teilweise, bevorzugt vollständig, mit der Hartstoffbeschichtung versehen ist.

3. Implantat nach Anspruch 1 oder 2, **dadurch gekennzeichnet, dass** die TiAIN-Außenschicht eine Zusammensetzung von Ti₁₋ₓAlₓN mit 0,2 ≤ x ≤ 0,8 aufweist, bevorzugt von 0,50 ≤ x ≤ 0,7, jeweils mit Ausnahme von unvermeidbaren Verunreinigungen.

4. Implantat nach einem der voranstehenden Ansprüche, **dadurch gekennzeichnet, dass** die TiAIN-Schicht einen Aluminium-Gradienten mit einem nach außen, von der Substratoberfläche in Richtung auf die Außenfläche des Implantats zunehmenden Aluminium-Anteil aufweist.

5. Implantat nach einem der voranstehenden Ansprüche, **dadurch gekennzeichnet, dass** die Grundschicht eine TiNbN-Schicht ist, besonders bevorzugt eine TiNbN-Schicht mit einer Zusammensetzung von Ti₁₋ₓNbₓN und 0,1 ≤ x ≤ 0,4, mit Ausnahme von unvermeidbaren Verunreinigungen.

6. Implantat nach einem der voranstehenden Ansprüche, **dadurch gekennzeichnet, dass** die Hartstoffbeschichtung eine Anbindungsschicht aus Titan oder einer Titanlegierung aufweist, die zwischen der Grundschicht und der Substratoberfläche vorgesehen ist, wobei die Anbindungsschicht eine Schichtdicke von 0 bis 500 nm aufweist.

7. Implantat nach einem der voranstehenden Ansprüche, **dadurch gekennzeichnet, dass** die Hartstoffbeschichtung eine Gesamtdicke in einem Bereich von 2 bis 10 µm aufweist, bevorzugt von 3 bis 8 µm und besonders bevorzugt von 4 bis 7 µm.

8. Implantat nach einem der voranstehenden Ansprüche, **dadurch gekennzeichnet, dass** die Grundschicht eine Schichtdicke in einem Bereich von 1 bis 4 µm aufweist.

9. Implantat nach einem der voranstehenden Ansprüche, **dadurch gekennzeichnet, dass** die TiAIN-Außenschicht eine Schichtdicke von 2 bis 5 µm aufweist.

10. Implantat nach einem der voranstehenden Ansprüche, **dadurch gekennzeichnet, dass** die Hartstoffbeschichtung einen oder mehrere der folgenden Eigenschaften aufweist:
eine mittlere Oberflächenrauheit Ra unterhalb von 0,05 µm, vorzugsweise unterhalb von 0,03 µm, und besonders bevorzugt in einem Bereich von 0,01 µm bis 0,03 µm;
eine Mikrohärte HV nach Vickers von 3200 bis 3600 HV bei Prüfkraft 0,080 N; und/oder
eine Rockwell-Haftfestigkeit nach DIN EN ISO 26443 von maximal Klasse 1.

11. Verfahren zur Herstellung eines Implantats gemäß einem der voranstehenden Ansprüche, welches die folgenden Schritte umfasst:
Bereitstellung eines Substrats aus einem metallischen Werkstoff, wobei das Substrat eine Oberfläche aufweist, und
Aufbringen einer Hartstoffbeschichtung auf die ganze oder einen Teil der Oberfläche mittels eines PVD-Verfahrens,
**dadurch gekennzeichnet, dass** das Aufbringen der Hartstoffbeschichtung dadurch erfolgt, dass eine Grundschicht aus TiN oder TiNbN auf die Substratoberfläche aufgebracht wird, und anschließend eine TiAIN-Schicht auf die Grundschicht aufgebracht wird, wobei die TiAIN-Schicht die Außenschicht der Hartstoffbeschichtung bildet.

## Claims

1. An implant for insertion into a human or animal body, comprising at least one substrate made of a metallic material and a surface formed on the substrate, the surface being provided in whole or in part with a hard material coating, **characterized in that** the hard material coating comprises a basic layer of TiN or TiNbN applied to the surface by a PVD process and an outer layer of TiAIN applied to the basic layer by a PVD process, the basic layer having a layer thickness in a range of from 1 to 5 µm and the TiAIN outer layer having a layer thickness of from 1 to 6 µm.

2. The implant according to claim 1, **characterized in that** the surface of the substrate is designed at least partly in the form of an artificial joint surface, the joint surface being provided in whole or in part, preferably completely, with the hard material coating.

3. The implant according to claim 1 or 2, **characterized in that** the TiAIN outer layer has a composition of Ti₁₋ₓAlₓN with 0.2 ≤ x ≤ 0.8, preferably of 0.50 ≤ x ≤ 0.7, in each case with the exception of inevitable impurities.

4. The implant according to any of the preceding claims, **characterized in that** the TiAIN layer has an aluminum gradient with an aluminum portion that increases outwards, from the substrate surface towards the outer surface of the implant.

5. The implant according to any of the preceding claims, **characterized in that** the basic layer is a TiNbN layer, particularly preferably a TiNbN layer having a composition of Ti₁₋ₓNbₓN and 0.1 ≤ x ≤ 0.4, with the exception of inevitable impurities.

6. The implant according to any of the preceding claims, **characterized in that** the hard material coating includes a bonding layer of titanium or a titanium alloy, which is provided between the basic layer and the substrate surface, the bonding layer having a layer thickness of from 0 to 500 nm.

7. The implant according to any of the preceding claims, **characterized in that** the hard material coating has a total thickness in a range of from 2 to 10 µm, preferably from 3 to 8 µm and particularly preferably from 4 to 7 µm.

8. The implant according to any of the preceding claims, **characterized in that** the basic layer has a layer thickness in a range of from 1 to 4 µm.

9. The implant according to any of the preceding claims, **characterized in that** the TiAIN outer layer has a layer thickness of from 2 to 5 µm.

10. The implant according to any of the preceding claims, **characterized in that** the hard material coating has one or more of the following properties:
a mean surface roughness Ra below 0.05 µm, preferably below 0.03 µm, and particularly preferably in a range of from 0.01 µm to 0.03 µm;
a microhardness HV according to Vickers of from 3200 to 3600 HV at a test load of 0.080 N; and/or
a Rockwell adhesive strength according to DIN EN ISO 26443 of class 1 at most.

11. A method of manufacturing an implant according to any of the preceding claims, comprising the following steps:
providing a substrate made of a metallic material, the substrate having a surface; and
applying a hard material coating to all or part of the surface by means of a PVD process,
**characterized in that** the hard material coating is applied by applying a basic layer of TiN or TiNbN to the substrate surface, and subsequently applying a TiAIN layer to the basic layer, the TiAIN layer forming the outer layer of the hard material coating.

## Revendications

1. Implant pour l'insertion dans un corps humain ou animal, comprenant au moins un substrat en un matériau métallique et une surface réalisée sur le substrat, la surface étant entièrement ou partiellement pourvue d'un revêtement de substance dure, **caractérisé en ce que** le revêtement de substance dure comprend une couche de base en TiN ou TiNbN appliquée sur la surface par un procédé PVD et une couche extérieure en TiAIN appliquée sur la couche de base par un procédé PVD, la couche de base présentant une épaisseur de couche dans une plage de 1 à 5 µm, et la couche extérieure de TiAIN présentant une épaisseur de couche de 1 à 6 µm.

2. Implant selon la revendication 1, **caractérisé en ce que** la surface du substrat est au moins partiellement réalisée sous forme de surface d'articulation artificielle, la surface d'articulation étant entièrement ou partiellement, de préférence complètement pourvue du revêtement de substance dure.

3. Implant selon la revendication 1 ou 2, **caractérisé en ce que** la couche extérieure de TiAIN présente une composition de Ti₁₋ₓAlₓN avec 0,2 ≤ x ≤ 0,8, de préférence 0,50 ≤ x ≤ 0,7, respectivement à l'exception d'impuretés inévitables.

4. Implant selon l'une des revendications précédentes, **caractérisé en ce que** la couche de TiAIN présente un gradient d'aluminium avec un taux d'aluminium augmentant vers l'extérieur, de la surface du substrat en direction de la surface extérieure de l'implant.

5. Implant selon l'une des revendications précédentes, **caractérisé en ce que** la couche de base est une couche de TiNbN, de manière particulièrement préférée une couche de TiNbN avec une composition de Ti₁₋ₓNbₓN et 0,1 ≤ x ≤ 0,4, à l'exception d'impuretés inévitables.

6. Implant selon l'une des revendications précédentes, **caractérisé en ce que** le revêtement de substance dure présente une couche de liaison en titane ou en un alliage de titane prévue entre la couche de base et la surface du substrat, la couche de liaison présentant une épaisseur de couche de 0 à 500 nm.

7. Implant selon l'une des revendications précédentes, **caractérisé en ce que** le revêtement de substance dure présente une épaisseur totale dans une plage de 2 à 10 µm, de préférence de 3 à 8 µm, et de manière particulièrement préférée de 4 à 7 µm.

8. Implant selon l'une des revendications précédentes, **caractérisé en ce que** la couche de base présente une épaisseur de couche dans une plage de 1 à 4 µm.

9. Implant selon l'une des revendications précédentes, **caractérisé en ce que** la couche extérieure de TiAIN présente une épaisseur de couche de 2 à 5 µm.

10. Implant selon l'une des revendications précédentes, **caractérisé en ce que** le revêtement de substance dure présente une ou plusieurs des propriétés suivantes :
une rugosité de surface moyenne Ra inférieure à 0,05 µm, de préférence inférieure à 0,03 µm, et de manière particulièrement préférée dans une plage de 0,01 µm à 0,03 µm;
une microdureté HV Vickers de 3200 à 3600 HV pour une force d'essai de 0,080 N; et/ou
une adhérence Rockwell selon DIN EN ISO 26443 de la classe 1 au maximum.

11. Procédé de fabrication d'un implant selon l'une des revendications précédentes, comprenant les étapes suivantes :
fournir un substrat en un matériau métallique, le substrat présentant une surface, et
application d'un revêtement de substance dure sur l'ensemble ou une partie de la surface par un procédé PVD,
**caractérisé en ce que** l'application du revêtement de substance dure est réalisée en appliquant une couche de base en TiN ou TiNbN sur la surface du substrat, et en appliquant ensuite une couche de TiAIN sur la couche de base, la couche de TiAIN formant la couche extérieure du revêtement de substance dure.
